# EUROPEAN PATENT APPLICATION

(11) **EP 4 410 766 A1**
(43) Date of publication of application: **07.08.2024**
(21) Application number: 23154298.6
(22) Date of filing: 31.01.2023
(51) Int. Cl.: C07C 29/141, C07C 31/20, C07C 29/78, C07C 45/72, C07C 47/19, C07C 29/80

(54) **PROCESS FOR PRODUCING 2-METHYL-1, 3-PROPANEDIOL AND SYSTEM**

(71) Applicant: ARKEMA FRANCE, 92700 Colombes (FR)
(72) Inventor: DUBOIS, Jean-Luc, 92705 COLOMBES CEDEX (FR)
(74) Representative: Arkema Patent

(57) **Abstract**

The present invention relates to a process for producing 2-methyl-1,3-propanediol.

In particular the present invention relates to process for producing 2-methyl-1,3-propanediol by performing an aldol reaction between propionaldehyde and formaldehyde to form 3-hydroxy-2-methylpropanal and hydrogenating of formed 3-hydroxy-2-methylpropanal.

The invention also relates to a system for producing 2-methyl-1,3-propanediol and more particularly a system for implementing a process for producing 2-methyl-1,3-propanediol

## Description

### [Field of the invention]

The present invention relates to a process for producing 2-methyl-1,3-propanediol.

In particular the present invention relates to process for producing 2-methyl-1,3-propanediol by performing an aldol reaction between propionaldehyde and formaldehyde to form 3-hydroxy-2-methylpropanal and hydrogenating of formed 3-hydroxy-2-methylpropanal.

The invention also relates to a system for producing 2-methyl-1,3-propanediol and more particularly a system for implementing a process for producing 2-methyl-1,3-propanediol.

### [Technical problem]

2-methyl-1,3-propanediol (MPDO) is a colorless, low viscosity and low toxicity liquid with a unique molecular structure. It is a branched aliphatic diol with two primary hydroxyl groups. This structure inhibits the crystallization of MPDO allowing it to remain liquid even at low temperatures (melting point is at Tₘ=-54°C).

The main applications are the manufacture of unsaturated polyester resin, saturated polyester, alkyd resin, polyurethane and plasticizer among others.

Today 2-methyl-1,3-propanediol is industrially produced, but mainly as a mixture and as coproduct, mostly with 1,4 butanediol.

One process is using propylene oxide as starting material for the production of 1,4 butanediol. After transformation to allyl alcohol followed by hydroformylation some 2-methyl-3-hydroxypropion aldehyde (HMPA) is formed along with 4-hydroxybutyraldehyde. During the subsequent hydrogenation, 2-methyl-1,3-propanediol is formed along with 1,4 butanediol.

As sustainable development might lead to other processes and methods for producing 1,4 butanediol and production units for 1,4 butanediol, there is a risk for the supply of 2-methyl-1,3-propanediol.

The new alternative ways for producing 1,4 butanediol are probably direct ways so that no 2-methyl-1,3-propanediol is coproduced anymore.

There is a need to produce 2-methyl-1,3-propanediol by alternative processes.

There is a need to produce 2-methyl-1,3-propanediol by a direct process not only as a by- or coproduct.

There is a need for a process of producing 2-methyl-1,3-propanediol as a single main product.

There is also a need for a process for producing 2-methyl-1,3-propanediol that avoids certain by-products, at least in larger amounts.

There is also a need of a process for producing 2-methyl-1,3-propanediol as a single product in a simple and easy process. By saying as a single product is meant that by-products make up less than 25 wt% of the product obtained directly after the performance of the process without any further purification step. In other words the 2-methyl-1,3-propanediol has a purity of at least 75%.

Another aim of the present invention is to propose a system for producing 2-methyl-1,3-propanediol as a single product.

### [BACKGROUND OF THE INVENTION]Prior art

The document US4,096,192 discloses a process for the preparation of 2-methyl-1,3-propanediol (MPD) by recycling mixed diols from the process for the preparation of 1,4-butanediol from acrolein and aliphatic diols until the final product is essentially 1,4-butanediol and MPD.

The document KR101738362 discloses a method for producing high purity 2-methyl-1,3-propanediol. The method comprises the steps of: making acrolein react with 1,3-butanediol to produce 2-methyl-1,3-propanediol and distilling the 2-methyl-1,3-propanediol containing the impurities in the presence of methylene chloride.

The document CN113845403 discloses a process for co-producing 2-methyl-1,3-propanediol and pentaerythritol. After a condensation reaction, a liquid containing 2-hydroxymethyl acrolein and trimethylolacetaldehyde is obtained. Said condensation reaction liquid is hydrogenated in a hydrogenation reactor by catalysis of a hydrogenation catalyst: 2-hydroxymethyl acrolein to 2-methyl-1,3-propanediol, and trimethylolacetaldehyde is hydrogenated to pentaerythritol.

None of the prior art discloses the new direct process.

### [Brief description of the invention]

Surprisingly it has been found that a process comprising the steps of:
(i) performing an aldol reaction between propionaldehyde and formaldehyde to form 3-hydroxy-2-methylpropanal,
(ii) hydrogenating of 3-hydroxy-2-methylpropanal.
allows to provide a process for producing 2-methyl-1,3-propanediol as a single product that possesses a sufficient purity.

Surprisingly it has been found that a system for producing 2-methyl-1,3-propanediol, said the system is comprising:
(a) a reactor R1 for performing an aldol reaction between propionaldehyde and formaldehyde to form 3-hydroxy-2-methylpropanal,
(b) a reactor R2 for hydrogenating 3-hydroxy-2-methylpropanal.
allows to implement a process for producing 2-methyl-1,3-propanediol as a single product that possesses a sufficient purity.

### [Brief description of drawings]

Figure 1 shows an example of the process (100) according to one embodiment of the invention. The essential steps are in rectangles with full line, the optional steps in rectangles with dashed line.
Figure 2 shows another example of the process (100) according to another embodiment of the invention. The essential steps are in rectangles with full line, the optional steps in rectangles with dashed line.
Figure 3 shows an example of a simplified flowsheet of one embodiment of the system comprising a reactor R1 for performing an aldol reaction between propionaldehyde and formaldehyde to form 3-hydroxy-2-methylpropanal and reactor R2 for hydrogenating 3-hydroxy-2-methylpropanal, as well as other elements as compressor C and separators S1 to S5.
Figure 4 shows another example of a simplified flowsheet of another embodiment of the system comprising a reactor R1 for performing an aldol reaction between propionaldehyde and formaldehyde to form 3-hydroxy-2-methylpropanal and reactor R2 for hydrogenating 3-hydroxy-2-methylpropanal, as well as other elements as separators S3 to S5.
Figure 5 shows a more detailed view of system of figure 3.
Figure 6 shows the side reactions that take place in the reactor R1 during the aldol reaction, yielding to methacrolein, 2-methyl-2-pentenal, 3-hydroxy-2-methyl-2hydroxymethylpropanal and propylpropiate.
Figure 7 shows the side reactions that take place in the reactor R2 during the hydrogenation, yielding to isobutanol and acetal between 3-hydroxy-2-methylpropanal and 2-methyl-1,3-propanediol and the hydrogenation of the products from the side reactions of the aldol reaction, yielding to 2-methallyl alcohol, 2-methyl-2-pent-1-ol, trimethylolethane and propanol.

### [Detailed description of the invention]

According to a first aspect, the present invention relates to a process (100) for producing 2-methyl-1,3-propanediol, said process is comprising the following steps:
(i) performing an aldol reaction (110) of propionaldehyde and formaldehyde to form 3-hydroxy-2-methylpropanal,
(ii) hydrogenating (140) the obtained 3-hydroxy-2-methylpropanal.

According to a second aspect, the present invention relates to a system for producing 2-methyl-1,3-propanediol and more particularly for implementing the process for producing 2-methyl-1,3-propanediol, the system is comprising:
(a) a reactor R1 for performing an aldol reaction between propionaldehyde and formaldehyde to form 3-hydroxy-2-methylpropanal,
(b) a reactor R2 for hydrogenating 3-hydroxy-2-methylpropanal.

By saying that a range from x to y in the present invention, it is meant that the upper and lower limit of this range are included, equivalent to at least x and up to y.

By saying that a range is between x and y in the present invention, it is meant that the upper and lower limit of this range are excluded, equivalent to more than x and less than y.

Optionally, according to specific embodiments, the process (100) of the present invention can comprise, in isolation or in any technically feasible combination, the following steps as well:
- providing propionaldehyde (102),
- providing formaldehyde (104),
- providing a catalyst C1 (106),
   combining or mixing (107a) the propionaldehyde and the formaldehyde
   combining or mixing (107b) the propionaldehyde, the formaldehyde and catalyst C1,
- adding (108) a catalyst C1 to the step of performing (110) the aldol reaction between propionaldehyde and formaldehyde,
- recovering(120) the catalyst C1
- recovering (125) unreacted propionaldehyde
- extracting (130) with an extraction column
- recovering (145) hydrogen by separation
- distilling (150)
- crystallizing (160).

The optional steps (102), (104), (106), (107a) or (107b) and (108) are performed before performing the aldol reaction (110).

The optional steps (120) and (130) are performed after performing the aldol reaction (110).

The step of performing the aldol reaction (110) between propionaldehyde and formaldehyde to form 3-hydroxy-2-methylpropanal is the following reaction:

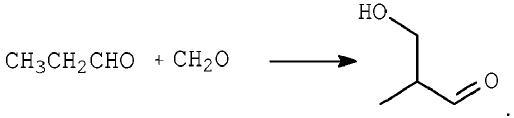

**As regards the step of performing** the aldol reaction (110) between propionaldehyde and formaldehyde to form 3-hydroxy-2-methylpropanal, it preferably takes place at a temperature between 40°C and 150°C, more preferably at a temperature between 50°C and 100°C and advantageously between 55°C and 80°C. Preferably, the pressure while performing the aldol reaction is between 2 bar and 20 bar, more preferably between 3 bar and 15 bar and advantageously between 3.5 bar and 10 bar.

Preferably, the propionaldehyde is in molar excess of formaldehyde. More preferably, the molar ratio between propionaldehyde and formaldehyde is between 1/1 and 2/1, more preferably 1.05/1 and 1.2/1.

Preferably, the step of performing the aldol reaction between propionaldehyde and formaldehyde to form 3-hydroxy-2-methylpropanal comprises additionally a catalyst C1. The catalyst C1 can be chosen from a tertiary amine. In a first preferred embodiment, the catalyst C1 is triethylamine. The quantity of the catalyst C1 used is preferably between 4wt% and 6wt% in view of the mass of the propionaldehyde.

According to a first preferred embodiment the step of performing the aldol reaction (110) is preceded by the steps (102), (104), (106) and (108) and subsequently followed by the steps (120) and (130) are performed.

According to a second preferred embodiment the step of performing the aldol reaction (110) is preceded by the steps (102), (104), (106) and (108).

The step of hydrogenating the obtained 3-hydroxy-2-methylpropanal is the following reaction:

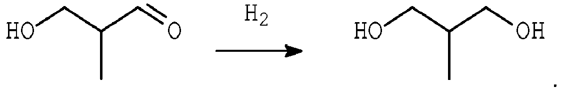

As regards the step of hydrogenating the obtained 3-hydroxy-2-methylpropanal, it preferably takes place at a temperature between 40°C and 150°C, preferably between 60°C and 130°C and advantageously between 80°C and 120°C. Preferably, the pressure while performing the hydrogenation is between 4 bar and 20 bar, preferably between 6 bar and 15 bar and advantageously between 8 bar and 12 bar.

In one embodiment the hydrogenation is continuous.

Preferably, the hydrogen is in molar excess of 3-hydroxy-2-methylpropanal. Preferably, the excess of hydrogen is relatively low, less than 100 %, preferably less than 50 %, more preferably less than 40 mol% excess. The molar excess of hydrogen towards 3-hydroxy-2-methylpropanal is at least 1%, preferably at least 2%.

Preferably, the step of hydrogenating the obtained 3-hydroxy-2-methylpropanal comprises also a catalyst C2. The catalyst C2 can be chosen from a transition metal based catalyst or an alloy transition metals. In a first preferred embodiment, the catalyst C2 is nickel based.

As regards the step of providing propionaldehyde (102) it can for example be made at 20°C (room temperature) and at 1bar.

As regards the step of providing formaldehyde (104) it can for example be made at 20°C and at 1 bar. The formaldehyde used can for example be in form of an aqueous solution with 37 wt% formaldehyde and 5 to 15 wt% methanol.

As regards the step of providing a catalyst C1 (106) it can for example be made at 20°C and at 1 bar. The catalyst C1 can be for example chosen from a tertiary amine, preferably tertiary aliphatic amines. In one embodiment, the catalyst C1 is triethylamine.

As regards the step of adding (108) a catalyst C1, it is preferably together with the other components or added last. The catalyst C1 can be mixed with the propionaldehyde and the formaldehyde shortly before performing the aldol reaction.

In one embodiment the mixing of catalyst C1 with the propionaldehyde and the formaldehyde is performed in a condenser.

In another embodiment the mixing of catalyst C1 with the propionaldehyde and the formaldehyde is performed by feeding lines that are combined.

As regards the step of recovering (125) the unreacted propionaldehyde after the step of performing (110) the aldol reaction, it is preferably made by distilling. The distillation is made under reduced pressure.

Preferably, the reduced pressure is between 0.1bar and 0.9 bar, advantageously between 0.3 bar and 0.5 bar.

As regards the step of recovering (120) the catalyst C1, if present, the catalyst C1 for the step of performing the aldol reaction can be recovered as well. As regards the step of recovering the catalyst C1 after the step of performing the aldol reaction, it is preferably made by distilling. The distillation is made under reduced pressure. Preferably, the reduced pressure is between 0.1 bar and 0.9 bar. In one embodiment the pressure is between 0.3 bar and 0.5 bar.

According to a first preferred embodiment the step of recovering the unreacted propionaldehyde and the catalyst C1 are made together under same conditions, by distillation.

As regards the step of extracting (130) with an extraction column, this step made to separate the components from recovering step (120). For instance, methanol coming from the aqueous solution with formaldehyde has been recovered by the distillation. The extraction can be a liquid-liquid extraction. In a first embodiment water is used for the extraction. In one embodiment the extraction is made at a temperature of 20°C at a pressure of 1 bar.

As regards the step of recovering (145) hydrogen that did not react in the hydrogenating step (140), it aims to recycle the hydrogen. The step comprises the separation of the two phases coming from the reactor R2, a gas phase comprising hydrogen and some impurities and a liquid phase comprising organic products, into the two respective phases. This separation takes place in separator S3. Preferably the separation is made under the same temperature and pressure conditions as during the hydrogenating step (140) in a liquid phase and a gaseous phase comprising the recovered hydrogen.

As regards the step of distilling (150) it is made to remove low boiling impurities and by-products. Preferably, the step of distilling (150) is performed under reduced pressure, in order to avoid having a too high temperature. This distillation takes place in separator S4, which is a distillation column. Preferably it takes place at a temperature between 60°C and 150°C, advantageously between 80°C and 130°C. Preferably, the pressure while performing the distillation is between 0.05 bar and 0.2 bar, advantageously between 0.05 bar and 0.15 bar.

As regards the step of crystallizing (160) it is made to separate impurities and by-products having a high melting point. Preferably the step of crystallizing (160) it made at a temperature between 10°C and 30°C, advantageously between 15°C and 25°C. Preferably, the pressure while performing the crystallization is between 0.9bar and 1.1 bar, advantageously between 0.95 bar and 1.05 bar. Optionally a solvent can be added before crystallization, that is a bad solvent for the impurities and by-products. This crystallization takes place in separator S5, which is a crystallizer or preferably a cascade of crystallizer.

The second aspect of the present invention relates to a system for producing 2-methyl-1,3-propanediol and more particularly for implementing the process (100) for producing 2-methyl-1,3-propanediol. Said system is comprising:
(a) a reactor R1 (20) for performing an aldol reaction between propionaldehyde and formaldehyde to form 3-hydroxy-2-methylpropanal, and
(b) a reactor R2 (50) for hydrogenating 3-hydroxy-2-methylpropanal.

Optionally, according to specific embodiments, the system of the present invention can comprise, in isolation or in any technically feasible combination, the following elements as well:
- a compressor C
- a separator S1,
- a separator S2,
- a separator S3,
- a separator S4,
- a separator S5, and
heat-exchanger(s), condenser(s), boiler(s) and connecting lines between the respective elements.

Preferably separator S1 is a distillation column.

Preferably separator S2 are means to perform a liquid-liquid extraction.

Preferably separator S3 are means to perform a gas-liquid separation.

Preferably separator S4 is a distillation column.

Preferably separator S5 are means to perform a crystallization.

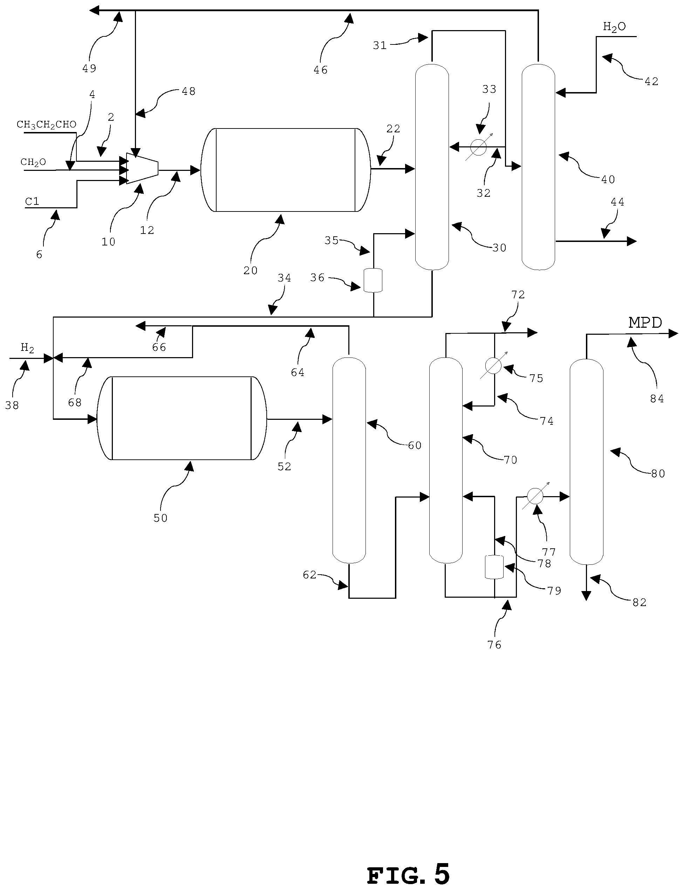

In a first exemplary embodiment, as shown in figure 5, the system comprises a compressor C (10), a reactor (20) for the aldol reaction, a separator S1 (30), a separator S2 (40), a reactor (50) for hydrogenating, a separator S3 (60), a separator S4 (70), a separator S5 (80), a feeding line (2) for propionaldehyde, a feeding line (4) for formaldehyde, a feeding line (6) for the catalyst C1, a connecting line (12) going from compressor C (10) to the reactor (20), a connecting line (22) going from the reactor (20) to separator S1 (30), a connecting line (31) going from separator S1 (30) to separator S2 (40), a connecting line (32) comprising a condenser (33) said connecting line (32) is going from connecting line (31) back to separator S1 (30), a connecting line (34) going from separator S1 (30) to reactor (50), a connecting line (35) comprising a boiler (36) said connecting line (35) is going from connecting line (34) back to separator S1 (30), a water feeding line (42) to separator S2 (40), an extraction line (44) from separator S2 (40), a line (46) for recycling raw materials (propionaldehyde and formaldehyde) from Separator S2 (40), said recycled raw materials could be reintroduced directly via line (48) or transferred via line (49) somewhere else, a H₂ feeding line (38), a connecting line (52) going from the reactor (50) to separator S3 (60), a connecting line (62) going from separator S3 (60) to separator S4 (70), a line (64) for recycling H₂ from Separator S3 (60), said recycled H₂ could be reintroduced directly via line (68) or transferred via line (68) somewhere else, an extraction line (72) from separator S4 (70), a connecting line (74) comprising a condenser (75) said connecting line (74) is going from extraction line (72) back to separator S4 (70), a connecting line (76) comprising a condenser (77) said connecting line (76) is going from separator S4 (70) to separator S5 (80), a connecting line (78) comprising a boiler (79) said connecting line (78) is going from connecting line (76) back to separator S4 (70), two extraction lines (82) and (84) from separator S5 (80).

In a second exemplary embodiment, the system comprises a reactor (20) for the aldol reaction, a reactor (50) for hydrogenating, a separator S3 (60), a separator S4 (70), a separator S5 (80), similarly to first exemplary embodiment, but contrary to first exemplary the embodiment, second exemplary embodiment does not comprise a compressor C (10), a separator S1 (30), a separator S2 (40) .

### [Figures]

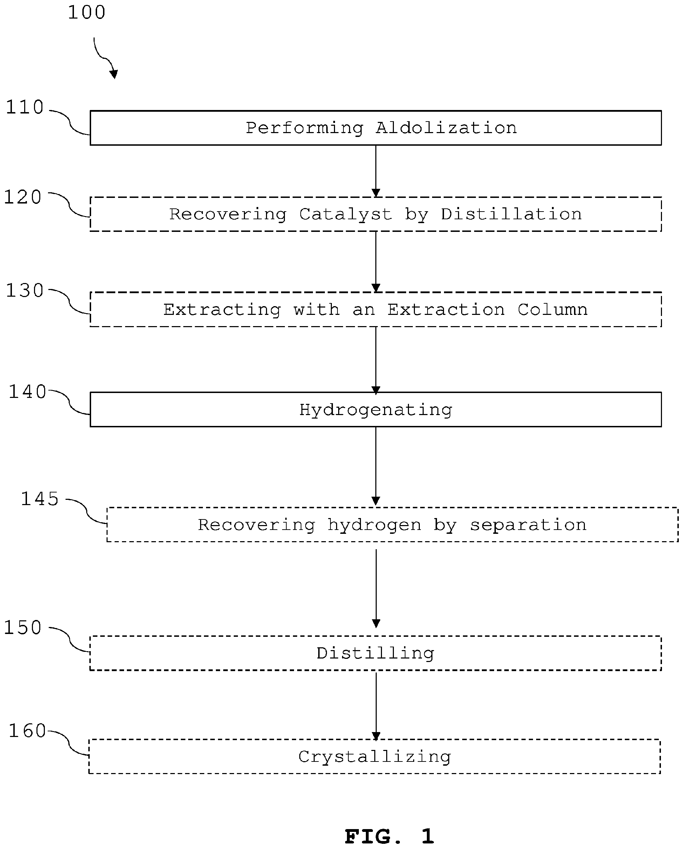

Figure 1 is according to a first preferred embodiment of the whole process (100).

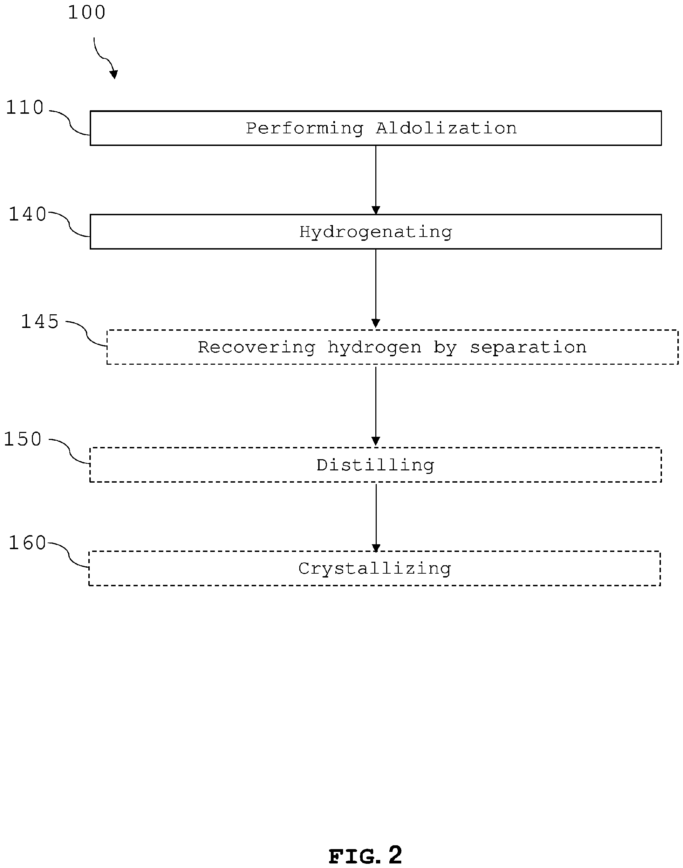

Figure 2 is according to a second preferred embodiment of the process (100) comprising less process steps than the first preferred embodiment.

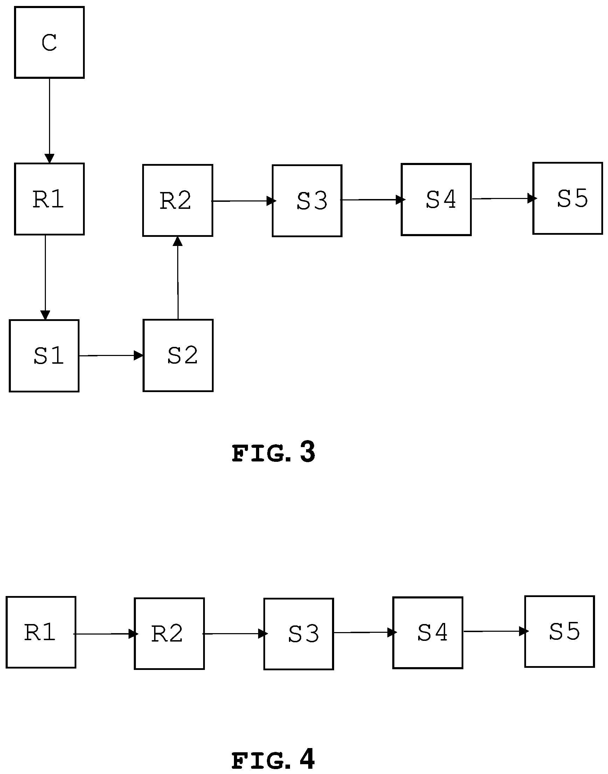

Figure 3 is a schematic representation of the system comprising the elements for the process according to the first preferred embodiment of the process (100) comprising essential and optional process steps.
Figure 4 is a schematic representation of the system comprising the elements for the process according to the second preferred embodiment of the process (100) comprising essential and optional process steps.

### [Methods]

The different components are analysed by gas chromatography, liquid chromatography, or chemical titration for example for conjugated double bonds.

The temperature at the different elements of the system is measured with temperature sensors.

The pressure at the different elements of the system is measured with pressure sensors.

### [Examples]

### Example 1

According to the first embodiment the process is performed with the system according the flowsheet of figure 3 which is shown more detailed in figure 5.

Formaldehyde in form of an aqueous solution at 37 wt% comprising as well 10 % of methanol is added to propionaldehyde at 20°C. Propionaldehyde is in molar excess of 10%.

Triethylamine is added as catalyst C1 at 5wt% of the propionaldehyde.

The three raw materials are added to compressor C and are transferred via line (12) at 31.4°C at 4bar to the reactor R1 (20) for performing the aldol reaction.

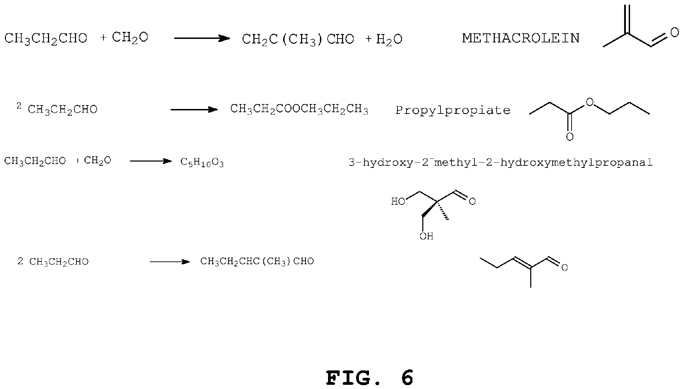

The aldol reaction between propionaldehyde and formaldehyde to form 3-hydroxy-2-methylpropanal take place in reactor R1 (20) at 60°C and a pressure of 4bar. The conversion of propionaldehyde is 0.9 and yield of the aldolization to form 3-hydroxy-2-methylpropanal is 0.791, other products being methacrolein with a yield of 0.001, 2-methyl-2-pentenal with a yield of 0.045, 3-hydroxy-2-methyl-2hydroxymethylpropanal with a yield of 0.018 and propylpropiate with a yield of 0.045. The reactions for the other products are detailed in figure 6.

The reaction mixture obtained in the reactor R1 (20) is transferred via line (22) at 60°C at 1 bar into the separator S1 (30), which is a distillation column. The distillation in separator S1 (30) takes place under reduced pressure at 0.38 bar at 60°C. The unreacted propionaldehyde, catalyst triethylamine and methanol are separated from the rest of the mixture via line (31).

Line (31) goes to separator S2 (40) in order to perform a liquid-liquid extraction. The liquid-liquid extraction if performed by adding water via line (42) in separator S2 (40). Extraction takes place at 20°C and 1bar. The methanol is removed with the water via line 44. The unreacted propionaldehyde, catalyst triethylamine can be recycled via lines (46) and (48) to the compressor C (10).

The reaction mixture that has been separated of unreacted propionaldehyde, catalyst triethylamine and methanol in separator S1 is transferred via line (34) to the reactor R2 (50), for hydrogenating the 3-hydroxy-2-methylpropanal obtained in the aldol reaction.

Hydrogen is added via line (38) at 20°C and 10 bar in small excess. As catalyst C2 for the hydrogenating step Raney Nickel is used.

The hydrogenating of 3-hydroxy-2-methylpropanal in reactor R2 (50) takes place at 100°C at 10 bar. The conversion of 3-hydroxy-2-methylpropanal is 0.99; the yield towards 2-methyl-1,3-propanediol 0.94; other products being isobutanol with a yield of 0.05 and an acetal between 3-hydroxy-2-methylpropanal and 2-methyl-1,3-propanediol with a yield of 0.001.

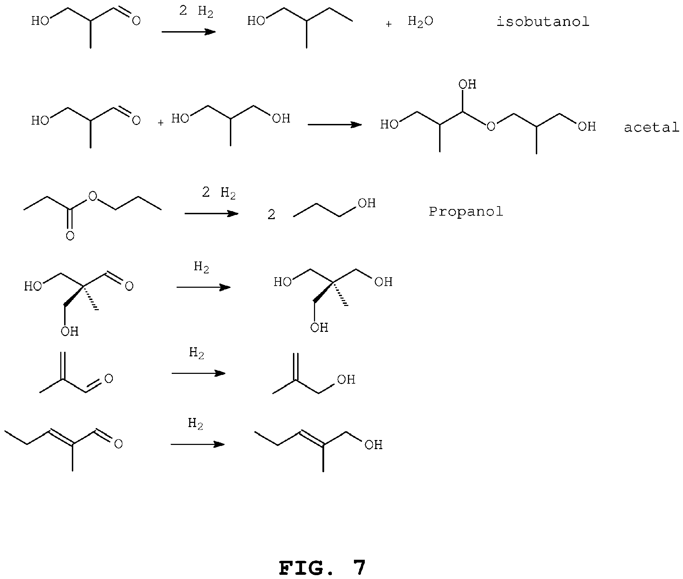

The other products obtained during the aldol reaction especially namely the 2-methyl-2-pentenal, the 3-hydroxy-2-methyl-2hydroxymethylpropanal and the propylpropiate are hydrogenated as well, yielding respectively to 2-methyl-2-pentenol, trimethylolethane and propanol, but also methacrolein yielding to 2-methallyl alcohol. The reactions for the other products are detailed in figure 7.

The reaction mixture obtained in the reactor R2 (50) is transferred via line (52) at 100°C at 10 bar into the separator S3 (60), which is a column to perform a gas liquid separation, in order to recover and recycle the excess hydrogen. The separation takes place under the same conditions as the hydrogenating reaction at 100°C and 10bar. Via line (64) the hydrogen is recycled and can be reintroduced via line (68).

Via line (62) the liquid phase containing all the organic products is transferred into separator S4 (70).

Separator S4 (70) is a distillation column. The low boiling parts from the liquid part of the hydrogenated reaction mixture is removed under reduced pressure at 0.1 bar at about 100°C. Via line (72) at 96°C essentially the 3-hydroxy-2-methylpropanal, 2-methyl-2-pentenol, propanol, isobutanol and the acetal, but also remaining water are separated from the rest. Via line (76) at 100°C and 1 bar, a liquid mixture comprising essentially 2-methyl-1,3-propanediol and trimethylolethane is coming from separator S4 (70) is transferred into separator S5 (80) and cooled down to 20°C with condenser (77) before entering said separator S5 (80).

Separator S5 (80) is a crystallizer. The 2-methyl-1,3-propanediol and trimethylolethane are separated by crystallization at 20°C at 1 bar. Trimethylolethane crystalizes at 20°C while 2-methyl-1,3-propanediol stays liquid. The two components are separated and the liquid 2-methyl-1,3-propanediol is recovered via line (84).

The overall conversion of propionaldehyde to 2-methyl-1,3-propanediol was 0.8; for 1kg of fed propionaldehyde 1.24 kg of 2-methyl-1,3-propanediol were obtained.

### Example 2

According to the second embodiment the process is performed with the system according the flowsheet of figure 4. This system does not comprise the compressor C (10), and the separators S1 (30) and separator S2 (40) and the respective lines connecting this missing equipment, and as well the recycling line (68) for hydrogen. There is direct connection line from reactor R1 (20) to reactor R2 (50). The operating conditions in the common equipment are the same as in example 1. In example 2, the consumption of the catalyst C1 triethylamine is much higher as it is not recovered: 4.3 times. In example 2, the consumption of the hydrogen is slightly higher as it is not recycled in the rector R2: 1.3 times.

The overall conversion of propionaldehyde to 2-methyl-1,3-propanediol was 0.77; for 1kg of fed propionaldehyde 1.20 kg of 2-methyl-1,3-propanediol were obtained.

## Claims

1. A process for preparing 2-methyl-1,3-propanediol, said process is comprising the following steps::
(i) performing an aldol reaction between propionaldehyde and formaldehyde to form 3-hydroxy-2-methylpropanal,
(ii) hydrogenating of 3-hydroxy-2-methylpropanal.

2. The process according to claim 1, **characterized in that** it comprises adding a catalyst C1 to the step of performing the aldol reaction between propionaldehyde and formaldehyde.

3. The process according to claim 1 or 2, **characterized in that** it further comprises the step of recovering the unreacted propionaldehyde.

4. The process according to any of any of claims 1 to 3, **characterized in that** the the propionaldehyde is in molar excess of formaldehyde.

5. The process according to any of any of claims 1 to 4, **characterized in that** the hydrogen is in molar excess of 3-hydroxy-2-methylpropanal.

6. The process according to any of any of claims 1 to 5, **characterized in that** it further comprises the step recovering the catalyst C1.

7. The process according to any of any of claims 1 to 5, **characterized in that** it further comprises the step recovering the hydrogen.

8. The process according to any of any of claims 1 to 7, **characterized in that** it further comprises the step of distilling.

9. The process according to claim 8, **characterized in that** the distilling takes place at a temperature between 60°C and 150°C.

10. The process according to any of any of claims 1 to 9, **characterized in that** it further comprises the step crystallizing.

11. The process according to claim 10, **characterized in that** the crystallizing is made at a temperature between 10°C and 30°C.

12. A system for producing 2-methyl-1,3-propanediol and for implementing the process according to claims 1 to 11, the system is comprising:
(a) a reactor R1 for performing an aldol reaction between propionaldehyde and formaldehyde to form 3-hydroxy-2-methylpropanal, and
(b) a reactor R2 for hydrogenating of 3-hydroxy-2-methylpropanal.

13. A system for producing 2-methyl-1,3-propanediol comprising:
(a) a reactor R1 for performing an aldol reaction between propionaldehyde and formaldehyde to form 3-hydroxy-2-methylpropanal, and
(b) a reactor R2 for hydrogenating of 3-hydroxy-2-methylpropanal.

14. The system according to claim 120 or 13, **characterized in that** said system comprises in isolation or in any technically feasible combination, the following elements as well
- a compressor C
- a separator S1 in form of a a distillation column,
- a separator S2 with means to perform a liquid-liquid extraction,
- a separator S3 with means to perform a gas-liquid separation,
- a separator S4 in form of a distillation column,
- a separator S5 with means to perform a crystallization.

15. The system according to to any of any of claims 12 to 14, **characterized in that** it comprises heat exchanger(s), condenser(s), boiler(s) and connecting lines between the respective elements.
